# EUROPÄISCHE PATENTSCHRIFT

(11) **EP 3 551 236 B1**
(45) Veröffentlichungstag und Bekanntmachung des Hinweises auf die Patenterteilung: **29.01.2025**
(21) Anmeldenummer: 17818080.8
(22) Anmeldetag: 06.12.2017
(51) Int. Cl.: A61L 2/26, G09F 3/20

(54) **SCHNITTSTELLE ZUR BEFESTIGUNG VON ANBAUTEILEN AN STERILCONTAINERN**
INTERFACE FOR FASTENING ATTACHMENT PARTS TO STERILE CONTAINERS
INTERFACE POUR FIXER DES ÉLÉMENTS RAPPORTÉS SUR DES CONTENEURS STÉRILES

(30) Priorität: 08.12.2016 DE 102016123864
(43) Veröffentlichungstag der Anmeldung: 16.10.2019
(73) Patentinhaber: Aesculap AG, 78532 Tuttlingen (DE)
(72) Erfinder: BOHNENSTENGEL, Philipp, 78256 Steißlingen (DE); HENKE, Matthias, 78567 Fridingen (DE); ZIERIS, Gerold, 78570 Mühlheim (DE); FRECH, Bozica, 78598 Königsheim (DE); MALIGLOWKA, Johann, 78600 Kolbingen (DE); STEINER, Sabrina, 78665 Frittlingen (DE); BAUER, Stephan, 78576 Emmingen (DE); MOTZ, Corvin, 88630 Pfullendorf (DE); AMANN, Joachim, 78357 Mühlingen - Zoznegg (DE); SCHEIT, Michael, 72770 Reutlingen (DE); HÖFLER, Martina, 78647 Trossingen (DE)
(74) Vertreter: Winter, Brandl - Partnerschaft mbB
(86) Internationale Anmeldenummer: PCT/EP2017/081701
(87) Internationale Veröffentlichungsnummer: WO 2018/104390

(56) Entgegenhaltungen:
- DE-C1- 19 703 823
- US-A- 4 026 033
- US-A1- 2006 162 210
- US-A1- 2013 175 276
- US-A1- 2014 091 089
- US-A1- 2014 259 836

## Beschreibung

Die vorliegende Erfindung betrifft einen Behälter bzw. Sterilcontainer, an dem ein Anbauteil befestigbar ist, und ein Behältersystem mit einem solchen Behälter.

Derartige Behälter oder Sterilcontainer finden beispielsweise in Krankenhäusern, Kliniken, Labors oder ähnlichen Einrichtungen zur Sterilisation von entsprechenden Gütern Verwendung. Zur Sterilisation der Güter werden diese in den Behälter eingelegt und gemeinsam mit diesem in den Sterilisator/Autoklaven eingelegt. Nach dem Sterilisationsvorgang kann der Sterilcontainer gemeinsam mit den darin befindlichen, nun sterilen Gütern aus dem Sterilisator entnommen werden und bietet bis zum Öffnen des Sterilcontainers eine Sterilbarriere für diese. Oftmals ist es nötig, Schnittstellen zum Anbringen von Anbauteilen an solchen Sterilcontainern vorzusehen, um beispielsweise Etiketten lösbar an der Außenwandung des Behälters festzulegen. Dabei ist es vor allem von Vorteil, wenn ein solches Befestigen/Lösen werkzeuglos erfolgen kann und wenige Handhabungsschritte seitens des Anwenders erfordert, ohne dass die Integrität der Sterilbarriere während des Vorgangs beschädigt oder gefährdet wird.

Zum Befestigen von Anbauteilen an Sterilcontainern der eingangs beschriebenen Art sind aus dem Stand der Technik verschiedene Lösungen bekannt. So werden Anbauteile üblicherweise an die Containerwanne genietet, wie z.B. in der DE 10 2009 006 427 A1 offenbart wird, in welcher ein Rahmenteil an einen Teil der Containerwanne genietet wird.

Eine solche Lösung hat den gravierenden Nachteil, dass die Containerhülle beim Nieten beschädigt/perforiert wird. Jede Beschädigung der Containerhülle muss dauerhaft verschlossen werden, um eine sichere Sterilbarriere zu gewährleisten. Darüber hinaus beinhaltet das Nieten mehrere Produktionsschritte. Zunächst müssen Löcher für die Nieten erzeugt und anschließend Nieten eingelegt und gepresst werden.

Ebenfalls sind aus dem Stand der Technik Lösungen bekannt, bei denen zunächst Halterungen an der Containerwanne befestigt werden, auf welche anschließend die Anbauteile, bspw. Kunststoffplatten, gesteckt werden. Aus der DE 10 2012 215 121 A1 ist eine solche Befestigungsvorrichtung mit Halterung zum Befestigen einer Zentrierhilfe zum Zentrieren des Containerdeckels bekannt. Ebenfalls bekannt ist die Befestigung von Anbauteilen durch Erzeugung von Hinterschnitten in Containerblechteilen, an welchen die Anbauteile dann mittels Hebeln, Riegeln, Federmechanismen etc. gehalten werden.

Ferner ist aus US 2006/162210 A1 eine Kartenhaltervorrichtung bekannt, die z.B. an der Außenseite eines Sterilisationsbehälters für medizinische Geräte angebracht werden kann und eine Karte hält. Die Kartenhaltervorrichtung hat einen im Allgemeinen ebenen Flächenabschnitt, der eine Karte aufnehmen und befestigen kann und zwei Befestigungsschenkel aufweist, die sich klammerartig von Enden des Flächenabschnitts aus erstrecken. Jeder der Befestigungsschenkel umfasst Kupplungselemente, welche Kupplungselemente des Sterilcontainers eingreifen. Die Kupplungselemente des Sterilcontainers werden durch Haken gebildet, die aus einer Wand des Containers ausgeschnitten und nach außen gebogen sind.

Ein in DE 197 03 823 C1 offenbarter Sterilcontainer hat einen Träger mit Öffnungen an den gegenüberliegenden Rändern zum Einsetzen und Entfernen einer Federklemme oder eines anderen Halters auf der anderen Seite des Containers. Alternativ hat der Träger eine Öffnung zum Durchführen einer Halterung. Der Behälter hat eine Federklammer mit Federbeinen auf der Vorder- und Rückseite, die eine Feder und ein Etikett bilden. Das Etikett ist mit einem elektronischen Datenträger und einem Träger versehen, der es an seinem Platz hält. Der vordere Federschenkel hat einen flachen Bereich mit einer Einführöffnung oder einem Halter zur Aufnahme des Etikettenträgers in einer Auslösevorrichtung. Die Klammeranordnung ist aus rostfreiem Federstahl und der Träger aus einem hitze-, säure- und druckbeständigen Kunststoff.

US 2014/091089 A1 offenbart eine medizinische Dokumentenbefestigungseinrichtung für einen medizinischen Sterilisierbehälter. Die Dokumentenbefestigungseinrichtung umfasst einen eine Oberseite aufweisenden Sterilisierbehälterdeckel und mindestens einen Dokumentenhalter, der einen ersten Befestigungsabschnitt und einen davon beabstandeten zweiten Befestigungsabschnitt umfasst sowie einen zwischen dem ersten Befestigungsabschnitt und dem zweiten Befestigungsabschnitt angeordneten Klemmabschnitt, wobei bei Verbindung des Dokumentenhalters mit dem Sterilisierbehälterdeckel der erste Befestigungsabschnitt und der zweite Befestigungsabschnitt am Sterilisierbehälterdeckel festgelegt sind und wobei zwischen dem Klemmabschnitt und der Oberseite des Sterilisierbehälterdeckels eine Dokumentenaufnahme angeordnet ist, in der ein Dokument mittels des Klemmabschnittes auf der Oberseite klemmend fixierbar ist.

Aus US 2013 0 175 276A1 ist ferner ein Sterilcontainer mit einer Wanne und einem Deckel bekannt, der in einer geschlossenen Position mittels Schnallen an der Wanne befestigt werden kann. An der Wanne ist ein vorspringendes Anbringungselement bereitgestellt, welches mit der Schnalle interagiert.

Die vorgenannten Lösungen haben allesamt den Nachteil, dass zum einen mehr Komponenten in der Produktion und zum anderen zusätzliche Schritte des Anwenders bei der Montage bzw. beim Fixieren des Anbauteils erforderlich sind (Hebel/Riegel umlegen/vorschieben). Befestigungslösungen, die aus mehreren Komponenten bestehen, sind zudem bezüglich ihrer Sterilisierbarkeit suboptimal, da sich in der Schnittstelle zwischen den Komponenten (z.B. Lagerbuchse eines Befestigungshebels) Verunreinigungen ansammeln können.

Es existieren auch Lösungen im Stand der Technik, bei denen Anbauteile am Sterilcontainer festgeschweißt oder anderweitig stoffschlüssig verbunden werden, allerdings ist ein Entfernen oder Austauschen der Komponenten bei einer solchen Lösung sehr aufwendig bzw. unmöglich.

Angesichts des vorstehend genannten Stands der Technik ist es die Aufgabe der vorliegenden Erfindung, ein gattungsgemäßes Behältersystem mit einem Behälter, insbesondere einem Sterilcontainer, und einem daran befestigbaren Anbauteil bereitzustellen. Ein bevorzugtes Ziel der vorliegenden Erfindung ist es dabei ein Behältersystem bzw. eine Schnittstelle bereitzustellen, das/die ohne die Behälterwandung zu perforieren eine, insbesondere werkzeuglos, lösbare Verbindung zwischen Behälter und Anbauteil ermöglicht.

Weiter macht es sich die vorliegende Erfindung zur bevorzugten Aufgabe, zur Vereinfachung der Herstellung, auf zusätzliche Komponenten für einen Verschlussmechanismus, wie zum Beispiel Riegel, Hebel und dergleichen, gänzlich zu verzichten.

Ein weiteres bevorzugtes Ziel der Erfindung ist es, die Handhabungsschritte, die seitens des Anwenders zum Anbringen und Lösen des Anbauteils am/vom Behälter nötig sind, so weit als möglich zu reduzieren.

Die vorstehend genannten Aufgaben und Ziele werden durch ein Behältersystem und einen Behälter mit den im Patentanspruch 1 bzw. nebengeordneten Anspruch angegebenen Merkmalen gelöst. Vorteilhafte Weiterbildungen sind Gegenstand der Unteransprüche.

Gemäß einem ersten Aspekt der Erfindung wird demzufolge einen Behälter in Form eines Sterilcontainers und ein zugehöriges Behältersystem vorgeschlagen, das den Sterilcontainer sowie ein am Behälter lösbar befestigbares Anbauteil aufweist. Erfindungsgemäß weist der Behälter dazu an seiner einen Behälterinnenraum definierenden Behälterwandung zumindest einen Befestigungsabschnitt bzw. Hinterschneidungsabschnitt auf, an welchem das Anbauteil formschlüssig befestigbar oder federelastisch arretierbar ist. Der Befestigungsabschnitt ist erfindungsgemäß stoffeinschlüssig mit einem geschlossenen, durchbruchfreien Abschnitt der Behälterwandung ausgebildet. Mit anderen Worten ist der Befestigungsabschnitt integral mit der Behälterwandung gefertigt, bzw. die Geometrie der Behälterwandung an sich bildet den Befestigungsabschnitt, so dass die Behälterwandung zum Erzeugen des Befestigungsabschnittes nicht durchbrochen/perforiert ist.

Die oben beschriebene integrale und durchgängige Fertigung des Befestigungsabschnittes mit der Behälterwandung bietet die Vorteile einer zuverlässigen Sterilbarriere und einer größtmöglichen Reduktion der Anzahl der zur Bereitstellung einer Befestigungsschnittstelle benötigten Komponenten seitens des Behälters.

Ferner ist eine Wandstärke des geschlossenen Abschnittes in der Behälterwandung, durch den der Befestigungsabschnitt gebildet wird, im Wesentlichen konstant und gleich der Wandstärke der an diesen Abschnitt angrenzenden Behälterwandung. Vorzugsweise kann dabei die Behälterwandung zumindest abschnittsweise aus einem Blech, vorzugsweise aus einem Blech aus einem für medizintechnische Anwendungen tauglichen Metall, besonders bevorzugt einem Aluminium, gebildet werden und zumindest ein Befestigungsabschnitt durch Blechumformen eines solchen Blechabschnitts gebildet sein.

Gemäß einer vorteilhaften Weiterbildung der Erfindung kann das Anbauteil zur Befestigung am Befestigungsabschnitt zumindest ein entsprechendes Befestigungsglied aufweisen, das von einer Befestigungsstellung, in welcher es zum Ausüben eines festlegenden Eingriffes mit dem entsprechenden Befestigungsabschnitt ausgebildet ist, in eine Auslenkungsstellung bzw. Überführungsstellung, in welcher das Befestigungsglied zum Überführen in den oder aus dem festlegenden Eingriff mit dem Befestigungsabschnitt ausgelenkt und/oder verformt ist, überführbar ist. Vorzugsweise kann das Anbauteil zwei Befestigungsglieder, also ein erstes und ein zweites Befestigungsglied aufweisen, welche in im Wesentlichen entgegengesetzte Richtungen auslenkbar/verformbar sind, um die Auslenkungsstellung einnehmen zu können.

Durch die Überführbarkeit des Befestigungsgliedes bzw. der Befestigungsglieder in eine Auslenkungsstellung, kann das Anbauteil in einen vollständigen Formschluss mit dem Befestigungsabschnitt gebracht und aus diesem wieder gelöst werden, ohne dass zusätzliche bewegliche Elemente, wie z.B. Riegel oder Hebel vorgesehen werden müssen. Eine solche Ausführungsform kann demnach für eine sichere Fixierung bei geringer Anzahl an erforderlichen Komponenten sorgen.

Gemäß einer bevorzugten Ausführungsform kann das zumindest eine Befestigungsglied oder das Anbauteil als Ganzes zumindest abschnittsweise federelastisch ausgebildet und dazu angepasst sein, durch die interne Federwirkung des zumindest einen federelastischen Abschnitts die Befestigungsglieder aus der Auslenkungsstellung in die Befestigungsstellung zurückzuführen. Eine elastische Ausführung des Anbauteils bzw. des Befestigungsgliedes ermöglicht ein Überführen zwischen Befestigungsstellung und Auslenkungsstellung, ohne dass zusätzliche Gelenke, Stelleinrichtungen oder dergleichen nötig sind, und stellt zudem sicher, dass das die Befestigungsglieder in der Befestigungsstellung vorgespannt sind, wenn das Anbauteil am Behälter befestigt ist.

Gemäß einem bevorzugten Ausführungsbeispiel kann der Befestigungsabschnitt und/oder das Anbauteil Führungsflächen aufweisen, welche derart abgeschrägt bzw. abgerundet sind, dass durch Aufdrücken des Anbauteils auf den Behälter in eine Befestigungsrichtung, vorzugsweise in eine Befestigungsrichtung im Wesentlichen senkrecht zu einer Behälterwand, das/die Befestigungsgliede(r), unter Abgleiten der Führungsflächen und Überwindung der internen Federwirkung, in die Auslenkungsstellung überführt werden. Vorzugsweise können die Führungsflächen zusätzlich dazu ausgebildet sein, durch eine symmetrische Anordnung das Anbauteil beim Aufdrücken automatisch bezüglich des Befestigungsabschnittes zu zentrieren.

Gemäß einer bevorzugten Ausführungsform kann der zumindest eine federelastische Abschnitt dazu angepasst sein, das/die Befestigungsgliede(r) in der Befestigungsstellung derart gegen den Befestigungsabschnitt anzustellen, dass es/sie eine klemmende Wirkung mit dem Befestigungsabschnitt entfaltet/entfalten. Anders ausgedrückt kann das Anbauteil bzw. das/die Befestigungsglied(er) dazu ausgebildet sein, eine kraft-/reibschlüssige Verbindung mit dem Befestigungsabschnitt zu erzeugen.

Bevorzugter Weise können zwei Befestigungsglieder derart mit dem Befestigungsabschnitt in formschlüssigen Eingriff bringbar sein, dass das Anbauteil in der Befestigungsstellung nur einen einzigen Freiheitsgrad relativ zum Behälter aufweist bzw. die formschlüssige Verbindung sich nur in einer Vorzugsrichtung lösen lässt. Dies hat den Vorteil, dass ein solcher Hinterschnitt, der sich in einer Vorzugsrichtung lösen lässt, fertigungstechnisch einfacher realisieren lässt, insbesondere bei Fertigungsverfahren wie z.B. Tiefziehen oder Spritzgießen, als ein vollständiger Formschluss. Gemäß einer besonders bevorzugten Ausführungsform kann ein Lösen in die Vorzugsrichtung durch eine kraftschlüssige Verbindung gemäß oben genanntem Aspekt unterbunden werden.

Die Ausführung der Behälterwanne von Sterilcontainern als Tiefziehteil aus Blech, insbesondere Aluminiumblech ist im Stand der Technik weit verbreitet. Darauf aufbauend kann gemäß einer bevorzugten Ausführungsform bei bereits existierenden Wannenkonzepten mittels Blechumformverfahren, wie z.B. Tiefziehen oder Formstanzen, der erfindungsgemäße Befestigungsabschnitt in die Behälterwandung eingebracht werden bzw. die Behälterwandung kann während des Tiefziehprozesses und/oder nachträglich so umgeformt werden, dass sie den Befestigungsabschnitt ausbildet. Dies bietet den zusätzlichen Vorteil, dass der Befestigungsabschnitt durch eine Sickenwirkung zusätzliche Steifigkeit/Stabilität in der Behälterwandung erzeugen kann.

Gemäß einem weiteren Aspekt der Erfindung kann bei einem Metallbehälter der Befestigungsabschnitt als kaltverformte Ausbuchtung (Vorsprung) oder Ausnehmung (Einrückung) in den Behälter eingebracht sein, ohne den Behälter zu durchbrechen.

Gemäß einem bevorzugten Ausführungsbeispiel kann der Befestigungsabschnitt an einer einzigen Behälterwand (Seitenwand, Boden oder Deckel), z.B. durch Umformen eines Blechs einer Behälterwand oder integrales Spritzgießen mit dieser, ausgebildet sein. Mit anderen Worten kann der Befestigungsabschnitt ausschließlich in einer Flächenebene eines Behälterwandungsabschnitts liegen/ ausgebildet sein. Dies hat den Vorteil, dass kleinere Anbauteile, wie z.B. Etikettenhalter an der Oberfläche einer einzigen Behälterwand anbringbar sind (im Gegensatz zu Lösungen, bei denen der Befestigungsabschnitt z.B. auf zwei gegenüberliegenden Behälterwänden liegt.

Gemäß einer weiteren bevorzugten Ausführungsform kann der Befestigungsabschnitt zumindest eine, insbesondere zwei, gemeinsame Symmetrieebenen mit dem/den Befestigungsgliedern aufweisen. Dies kann, insbesondere in Kombination mit dem Merkmal der Führungsflächen, eine automatische Zentrierung des Anbauteils unterstützen und sorgt zudem, im Falle einer kraftschlüssigen Verbindung, für eine gleichmäßige Krafteinleitung. Zudem sind Anbauteile, deren genaue Orientierung irrelevant ist, bei einer solchen Ausführungsform unabhängig von ihrer (z.B. vertikalen) Orientierung aufclipsbar.

Gemäß einem bevorzugten Ausführungsbeispiel kann das zumindest eine Befestigungsglied einstückig mit dem Grundkörper des Anbauteils ausgebildet sein bzw. kann das Anbauteil auch ganz einstückig gefertigt sein, vorzugsweise aus einem elastischen Material. Dies sorgt auch seitens des Anbauteils für eine größtmögliche Reduktion der Verbindungselemente und vereinfacht das Handling für den Anwender, da sowohl der Behälter, als auch das Anbauteil durch ihre ihnen eigene Geometrie und Materialeigenschaften ein Klicksystem zur werkzeuglosen und lösbaren Montage realisieren. Vorzugsweise ist das Anbauteil aus medizintechnischen und insbesondere sterilisationsbeständigen Materialien gefertigt, z.B. geeigneten Metallen oder Kunststoffen. Insbesondere kann für das Anbauteil ein hitzebeständiges Material verwendet werden. Besonders Bevorzugt kann das Befestigungsteil dazu angepasst und ausgelegt sein, mit den Standardprozessen der Aufbereitungseinheit für Medizinprodukte (AEMP) bzw. der zentralen Sterilgutversorgungsabteilung (ZSVA) thermisch gereinigt, chemisch gereinigt, im Reinigungs- und Desinfektionsgerät (RDG) gereinigt und in Sterilisationsprozessen Dampf-, Ethylenoxid- oder H2O2-sterilisiert zu werden.

Gemäß einer weiteren Ausführungsform kann der Befestigungsabschnitt in einem geschlossenen Wandungsabschnitt eine Art Tasche oder Schienen bzw. eine Kulissenführung ausbilden, in welche das Anbauteil direkt oder indirekt hineingelegt oder hineingeschoben werden kann. Vorzugsweise kann das Anbauteil bei einer solchen Ausführungsform mittels einer internen Klemmwirkung des Anbauteils oder einem beim Einführen zu überwindenden Noppen in der Tasche oder der Kulissenführung arretiert werden.

Gemäß einem weiteren Aspekt kann das Anbauteil zum abstützenden Kontakt mit der Behälterwand anstatt einer Auflagefläche eine Anzahl von Auflagerippen an seiner Rückseite aufweisen.

Gemäß einer bevorzugten Ausführungsform kann das Anbauteil im angebrachten Zustand einen Reinigungsspalt zum Container ausbilden.

Gemäß einem weiter bevorzugten Aspekt kann das Anbauteil eine Klammer, vorzugsweise eine Drahtklammer zum Befestigen eines Etiketts aufweisen.

Gemäß einem weiteren Aspekt kann das Anbauteil Löcher/Ausnehmungen zur Befestigung von Kennzeichnungsschildern aufweisen.

Gemäß einem weiteren Aspekt der Erfindung können die äußersten Endabschnitte des Anbauteils in Richtung von der Behälterwand weg gekrümmt sein, um ein Untergreifen zu erleichtern.

Die nachfolgende Beschreibung bevorzugter Ausführungsformen dient im Zusammenhang mit den Figuren der näheren Erläuterung der Erfindung. Die Figuren sind dabei lediglich schematischer Natur und dienen ausschließlich dem Verständnis. Die gleichen Elemente sind mit denselben Bezugszeichen versehen. Sofern nicht anders beschrieben, sind die unterschiedlichen Ausführungsbeispiele prinzipiell gleich aufgebaut sowie gleich funktionierend, wodurch die unterschiedlichen Merkmale der Ausführungsbeispiele frei miteinander kombinierbar sind.

Es zeigen:
- Fig. 1: einen Behälter/Sterilcontainer mit daran fixiertem Anbauteil in einer ersten Ausführungsform;
- Fig. 2: ein Detail eines Befestigungsabschnitts und eines Befestigungsglieds der ersten Ausführungsform in einem getrennten Zustand;
- Fig. 3: ein Detail des Befestigungsabschnitts und des Befestigungsglieds der ersten Ausführungsform in einem verbundenen Zustand;
- Fig. 4: ein Detail des Befestigungsabschnitts der ersten Ausführungsform;
- Fig. 5: einen Behälter/Sterilcontainer mit daran fixiertem Anbauteil in einer zweiten Ausführungsform;
- Fig. 6: ein Detail des Befestigungsabschnitts und des Befestigungsglieds der zweiten Ausführungsform in einem verbundenen Zustand;
- Fig. 7: einen Behälter/Sterilcontainer mit daran fixiertem Anbauteil in einer dritten Ausführungsform;
- Fig. 8: ein Detail des Befestigungsabschnitts und des Befestigungsglieds der dritten Ausführungsform in einem verbundenen Zustand;
- Fig. 9: einen perspektivischen Schnitt durch einen Behälter/Sterilcontainer mit daran fixiertem Anbauteil in einer vierten Ausführungsform;
- Fig. 10: das Anbauteil gemäß der vierten Ausführungsform;
- Fig. 11: das Anbauteil der vierten Ausführungsform im am Behälter/Sterilcontainer befestigten Zustand;
- Fig. 12: eine Schnittansicht des Behälters/Sterilcontainers mit daran fixiertem Anbauteil der vierten Ausführungsform; und
- Fig. 13: eine Ansicht der Rückseite des Anbauteils der vierten Ausführungsform.

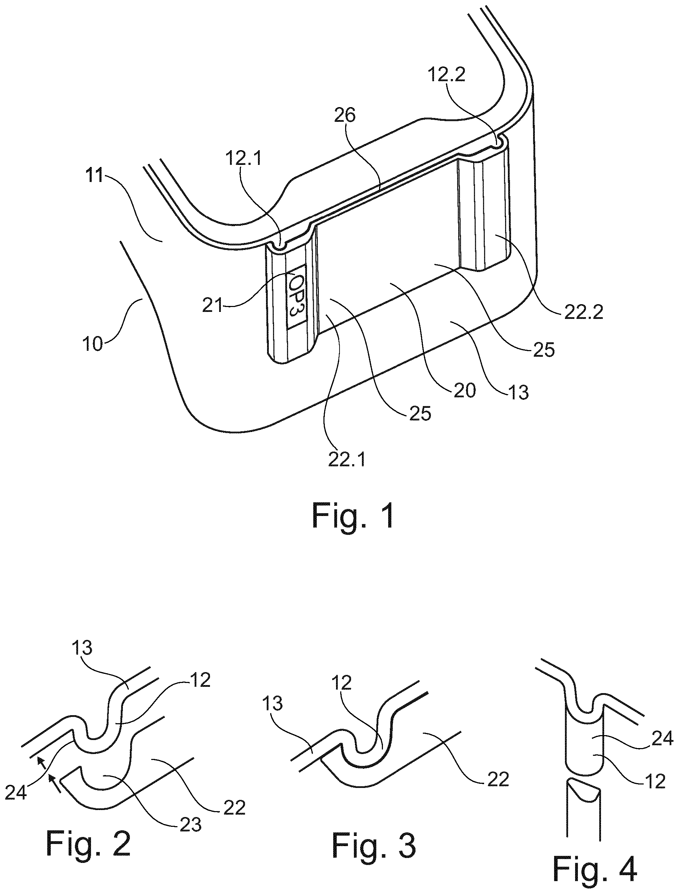

In Fig. 1 ist eine Teilansicht eines erfindungsgemäßen Behälters 10, bzw. eines wannenförmigen Unterteils eines erfindungsgemäßen Behälters 10, in einem ersten Ausführungsbeispiel dargestellt. Der Behälter 10 ist hierbei als ein Sterilisationsbehälter ausgeführt, der insbesondere zur Aufnahme, d.h. zur Aufbewahrung und zum Transport von medizinischen Utensilien, etwa chirurgischen Werkzeugen / Besteck oder anderen wiederverwendbaren medizinischen Gütern vorgesehen ist. Nach dem Sterilisiervorgang des in dem Behälter 10 enthaltenen Werkzeugs / der entsprechenden Utensilien, dient der Sterilisationsbehälter auch als Sterilbehälter, zur Aufbewahrung sowie zum Transport zwischen der Sterilisationsstelle und dem im Krankenhaus vorgesehenen Operationsbereich, etwa dem Operationssaal.

Da der Behälter 10 nicht geöffnet werden kann, ohne eine Kontamination des Inhalts zu riskieren, ist es wichtig, den Inhalt des Behälters 10, seinen Bestimmungsort (z.B. dem Operationssaal), den Zeitpunkt der Sterilisation etc. gut sichtbar an der Behälteraußenseite zu deklarieren. Zu diesem Zweck ist an dem gezeigten Behälter 10 ein als eine Art geschwungene Platte ausgeführtes, clipförmiges Anbauteil 20 gemäß der ersten Ausführungsform befestigt, welches zum Markieren des Behälters 10 bzw. als Halterung für Etiketten/Indikatorschilder 21 vorgesehen ist.

Erfindungsgemäß ist es vorgesehen, dass sich besagtes Anbauteil 20 per Hand an einer Behälter(außen)wandung 11 über eine, als eine Art Klick-System ausgeführtes Behältersystem bzw. Schnittstelle/Befestigungsvorrichtung "aufclipsen" und wieder entfernen lässt. Das gezeigte Behältersystem basiert grundlegend auf dem Zusammenwirken von (hier) zwei am Anbauteil 20 vorgesehenen Befestigungsgliedern 22 (erstes Befestigungsglied 22.1 und zweites Befestigungsglied 22.2), welche mit einem in/an der Behälterwandung 11 (im gezeigten Beispiel in einer einzigen Behälterwand 13) vorgesehenen Befestigungsabschnitt 12 in Eingriff bringbar sind, um somit das Anbauteil 20 am Behälter 10 festzulegen. Der Befestigungsabschnitt besteht im dargestellten Beispiel aus zwei Unterabschnitten/Hinterschneidungsstellen 12.1, 12.2, die Anzahl der Unterabschnitte/Hinterschneidungsstellen kann aber erfindungsgemäß variiert werden. Das Zusammenwirken der Befestigungsabschnitte 12 mit den Befestigungsgliedern 22 ist in den Figuren 2 bis 4 im Detail dargestellt. Die Befestigungsabschnitte 12 sind hier als nach außen vorstehende Vorsprünge/Protrusionen bzw. Verwölbungen in der Behälterwand 13 ausgeführt, welche Profile mit Hinterschnitt bilden, die sich abschnittsweise in vertikaler Richtung der Behälterwand 13 (orthogonal zum Behälterboden) an derselben erstrecken. Die Profile der Befestigungsabschnitte 12 sind in etwa hakenförmig ausgebildet, wobei die Öffnungen ihrer Hakenformen in entgegengesetzte Richtungen (voneinander abgewandt) zeigen, da die beiden Befestigungsabschnitte 12 im Wesentlichen symmetrisch bezüglich einer Symmetrieebene ausgebildet sind, die sich ebenfalls in Vertikalrichtung der Behälterwand 13 erstreckt. Im gezeigten Beispiel sind die Befestigungsabschnitte 12 durch Umformen der Behälterwand 13 ausgebildet, weshalb sie im Querschnitt über den Verlauf des Hakenprofils hinweg im Wesentlichen dieselbe Wandstärke aufweisen.

Die am Anbauteil 20 vorgesehenen Befestigungsglieder 22 sind im gezeigten Beispiel an gegenüberliegenden Randabschnitten des in etwa plattenförmigen Anbauteils 20 angeordnet und weisen an ihrem äußeren Rand ebenfalls eine Hakenform auf, deren Hakenöffnung 23 als komplementär zu den Befestigungsabschnitten 12 ausgebildete Nut/ rinnenförmige Vertiefung/Ausnehmung gestaltet ist.

Die erfindungsgemäße Schnittstelle/Befestigungsvorrichtung zwischen Behälter 10 und Anbauteil 20 ist also prinzipiell ähnlich einer Nut-Feder-Verbindung ausgebildet, bei der jedoch das Anbauteil 20 bei der gezeigten Ausführungsform nicht in Längsrichtung der Nut/Feder-Geometrie aufgeschoben wird, sondern stattdessen in eine Richtung senkrecht zur Behälterwand 13 aufgeclipst werden kann. Um ein solches Klick-System zu ermöglichen, ist eine gewisse Eigenelastizität des Anbauteils 20 bzw. der Befestigungsglieder 22 erforderlich, damit die Befestigungsglieder 22 zum Überwinden des hakenförmigen Hinterschnitts der Befestigungsabschnitte 12, in diesem Fall nach außen, ausgelenkt werden können bzw. sich derart verformen können (einen Auslenkungszustand einnehmen können), dass sich die Öffnung ihrer Hakenform vergrößert um den Hinterschnitt passieren zu können. Dazu ist das Anbauteil 20, vorzugsweise einstückig, aus einem Material mit der erforderlichen Elastizität sowie der nötigen Dauerfestigkeit gegen die Belastungen der Sterilisationszyklen (Druck, Feuchtigkeit, Temperatur) gefertigt, z.B. einem geeigneten Kunststoff oder einem geeigneten Metall (Legierung).

Weiter sind im gezeigten Beispiel sowohl die Befestigungsabschnitte 12 als auch die Befestigungsglieder 22 mit Führungsflächen 24 versehen, welche dazu ausgebildet sind ein aufclipsen zu unterstützen, indem sie, wenn das Anbauteil 20, in Richtung senkrecht zur Flächenebene der Behälterwand 13, auf diese gedrückt wird, derart aufeinander abgleiten, dass die Befestigungsglieder 22 in den Auslenkungszustand ausgelenkt oder verformt werden und das Anbauteil zudem automatisch zentriert wird. Zum Zwecke der automatischen Zentrierung weisen Befestigungsabschnitte 12 und Befestigungsglieder 22 im gezeigten Beispiel zudem gemeinsame Symmetrieebenen in vertikaler und in horizontaler Richtung (bezüglich des Behälters 10) auf.

Die Befestigungsglieder 22 des beispielhaft dargestellten Anbauteils 20 sind durch zur Behälteraußenseite hin konkav gebogene/geschwungene Abschnitte 25 an dasselbe angebunden, welche zum Erleichtern einer Auslenkung der Befestigungsglieder 22 in Krümmungsrichtung und damit auch zum Erleichtern einer Entnahme aus der Befestigungsstellung dienen. Die Oberfläche der Behälterwand 13, an welcher das Anbauteil 20 befestigt ist, ist an die so geschwungene Geometrie des Anbauteils 20 angepasst, was dazu führt, dass sich mittig eine Auflagefläche 26 bildet, welche bei einem Auslenken der Endabschnitte des Anbauteils 20, in eine Richtung von der Behälterwand weg, als abstützendes Gegenlager dienen kann, um das Lösen der Befestigungsglieder 22 aus den Befestigungsabschnitten 12 zu unterstützen.

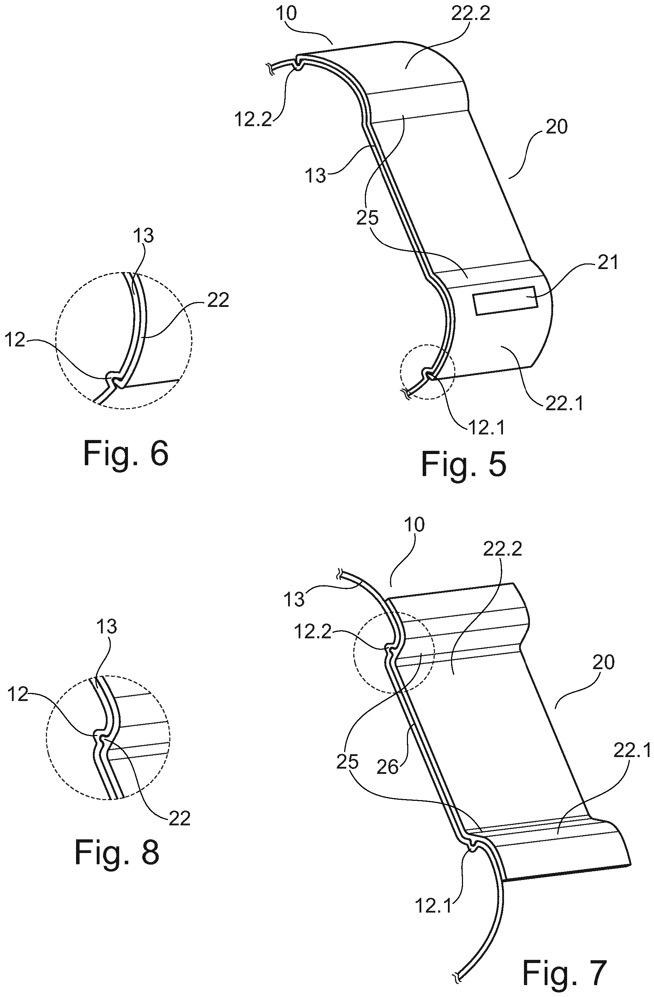

In Fig. 5 ist ein Behälter 10 mit daran befestigtem Anbauteil 20 gemäß einer zweiten Ausführungsform dargestellt, Fig. 6 zeigt eine Detailansicht des Befestigungsgliedes 22, welches sich in Eingriff mit dem Befestigungsabschnitt 12 des Behälters 10 gemäß der zweiten Ausführungsform befindet.

Das zweite Ausführungsbeispiel ist weitestgehend entsprechend dem ersten Ausführungsbeispiel gestaltet, mit dem Unterschied, dass die in der Behälterwand 13 ausgebildeten Befestigungsabschnitte 12 als rinnenförmige Vertiefungen ausgebildet und die Befestigungsglieder 22 als gegeneinander angestellte, in etwa hakenförmige Vorsprünge an gegenüberliegenden Endabschnitten des Anbauteils 20 ausgeführt sind. Die Nut/Feder-Beziehung ist, im Vergleich zum ersten Ausführungsbeispiel, in diesem Fall also invers ausgeführt.

In Fig. 7 ist ein Behälter 10 mit daran befestigtem Anbauteil gemäß einer dritten Ausführungsform dargestellt, Fig. 8 zeigt eine Detailansicht des Befestigungsgliedes 22 welches sich in Eingriff mit dem Befestigungsabschnitt 12 des Behälters 10 gemäß der dritten Ausführungsform befindet.

Bei der dritten gezeigten Ausführungsform sind die, hier wieder nut-artig bzw. konvex ausgebildeten, Befestigungsglieder 22 nicht an gegenüberliegenden Endabschnitten des Anbauteils 20, sondern nach Innen versetzt angeordnet (von den Endabschnitten beabstandet). Die Befestigungsglieder 22 sind im gezeigten Beispiel an der konvexen Seite der gebogenen Abschnitte 25 angeordnet, wodurch sich das Anbauteil 20 leicht vom Behälter lösen lässt, indem die Endabschnitte ergriffen und, unter Ausnutzung ihres Hebelarmes zu den gebogenen Abschnitten 25 und des abstützenden Kontakts der Auflagefläche 26 mit der Behälterwand 13, so ausgelenkt werden, dass sich die Krümmung der gebogenen Abschnitte 25 verstärkt und die Befestigungsglieder 22 aus ihrem Eingriff mit den Befestigungsabschnitten 12 gelöst werden.

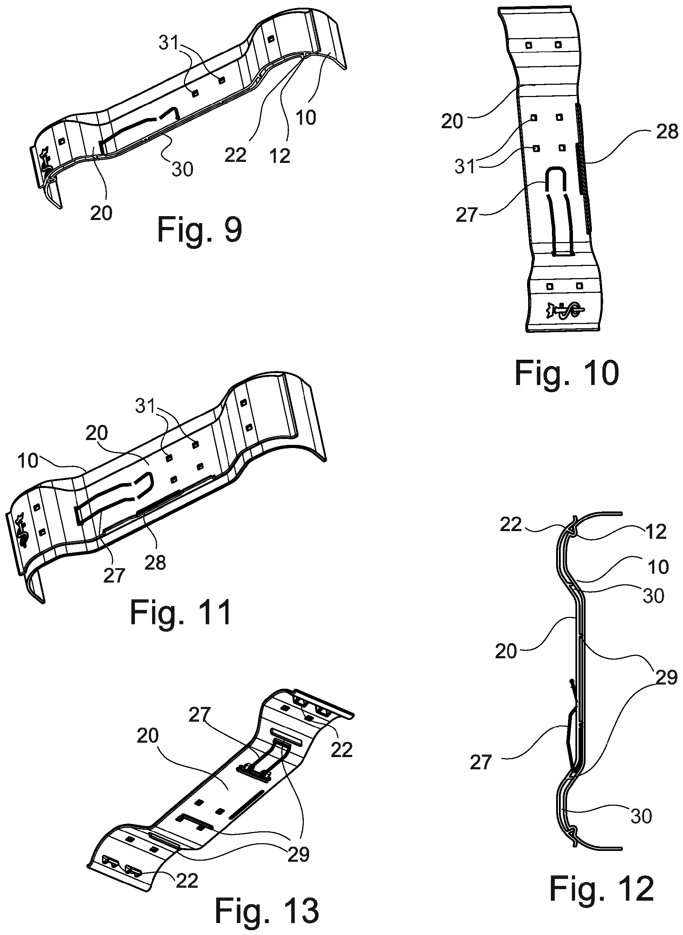

In den Fign. 9 bis 13 ist ein erfindungsgemäßes Anbauteil 20 gemäß einer vierten Ausführungsform abgebildet. Das Anbauteil 20 der vierten Ausführungsform ähnelt vom Grundprinzip dem zweiten Ausführungsbeispiel und wird ebenfalls über gegeneinander angestellte, in etwa hakenförmige Befestigungsglieder 22 in rinnenartigen Befestigungsabschnitten am Behälter 10 festgelegt. Im Gegensatz zum zweiten Ausführungsbeispiel sind die Befestigungsglieder 22 jedoch nicht an den äußersten Endabschnitten des Anbauteils 20 sondern jeweils etwas von der Kante nach innen versetzt angeordnet. Die äußersten Endabschnitte des Anbauteils 20 krümmen sich dafür von der Behälterwand 13 weg, wodurch ein erleichtertes Untergreifen und damit Entfernen des Anbauteils 20 vom Behälter 10 durch den Anwender ermöglicht wird.

Die vierte Ausführungsform offenbart zudem ein alternatives Konzept zum Befestigen eines Etiketts/Indikatorschilds 21 am Anbauteil 20. Dieses beinhaltet eine Drahtklammer 27, in Verbindung mit einer Fläche der Vorderseite des Anbauteils 20 eine klemmende Wirkung auf ein Etikett 21 ausüben zu können. Die Klammer 27 ist im gezeigten Beispiel in der Rückseite des Anbauteils 20 verrastet und wird durch eine Ausnehmung im Anbauteil hindurch zur Vorderseite geführt. Zur zusätzlichen Sicherung und zum Ausrichten eines befestigten Etiketts 21, weist das gezeigte Beispiel zudem einen unteren Anschlag 28 an der zur Befestigung des Etiketts 21 vorgesehenen Fläche auf.

Im Gegensatz zu den anderen Ausführungsbeispielen weist die vierte Ausführungsform keine Auflagefläche 26 auf. Stattdessen sind mehrere Auflagerippen 29 vorgesehen. Diese definieren einen Reinigungsspalt 30 mit einer vorbestimmten Spaltbreite, die dazu ausreicht, den Zwischenraum zwischen dem Anbauteil 20 und dem Behälter 10 zu reinigen bzw. zu sterilisieren. Zudem schafft der Spalt 30 Raum für die hinter dem Anbauteil geführte Drahtklammer 27. Das in den Figuren 9 bis 13 gezeigte Anbauteil 20 weist zudem Ausnehmungen bzw. Löcher 31 auf, welche zur Befestigung von Kennzeichnungsschildern mit entsprechenden komplementären (Rast-)Vorsprüngen ausgebildet sind.

Ausgehend von den gezeigten Ausführungsbeispielen ist eine Vielzahl an Varianten der vorliegenden Erfindung denkbar.

So kann die Anzahl der Unterabschnitte des Befestigungsabschnitts 12 bzw. dessen Gestaltung nahezu beliebig variiert werden (bspw. ein als einzelner runder Noppen ausgeführter Befestigungsabschnitt 12, an welchem das Anbauteil 20 arretierbar ist). Es ist zudem nicht notwendig, dass das Anbauteil 20 Befestigungsglieder 22 aufweist. Erfindungsgemäß sind auch andere Vorrichtungen denkbar, um das Anbauteil 20 am Befestigungsabschnitt 12 festzulegen. Z.B. könnte der Befestigungsabschnitt eine Art Tasche bilden, in welche das Anbauteil 20 hineingelegt oder hineingeschoben werden und z.B. mittels einer internen Klemmwirkung des Anbauteils 20 arretiert kann. Auch könnte der Befestigungsabschnitt 12 Schienen bzw. eine Kulissenführung ausbilden, in welche ein Abschnitt des Anbauteils 20 eingeschoben werden kann. Die Arretierung könnte in einem solchen Fall z.B. über einen Noppen hergestellt werden, welche am Behälter 10 oder am Anbauteil 20 vorgesehen ist.

Auch müssen die Behälterwandung 11 und der darin ausgebildete Befestigungsabschnitt 12 nicht aus einem Blech geformt sein, sondern könnte z.B. auch einstückig aus Kunststoff durch Spritzgießen hergestellt sein.

Prinzipiell ist es auch denkbar, anstatt der Befestigungsglieder 22 den Befestigungsabschnitt so auszulegen, dass er von der Befestigungsstellung in die Auslenkungsstellung überführbar ist, jedoch ist es, zumindest bei einer Anwendung im Bereich der Sterilcontainer, von Vorteil, wenn sich die Behälterwandung möglichst wenig verformt, um die Sterilbarriere zwischen Deckel und Containerwanne nicht zu gefährden.

Das Anbauteil 20 kann grundsätzlich auch rein formschlüssig oder rein kraftschlüssig am Behälter festgelegt sein.

### Bezugszeichen

- 10: Behälter/Sterilcontainer;
- 11: Behälterwandung;
- 12: Befestigungsabschnitt;
- 13: Behälterwand;
- 20: Anbauteil;
- 21: Etikett/Indikatorschild;
- 22: Befestigungsglied;
- 23: Hakenöffnung/Nut;
- 24: Führungsfläche;
- 25: gebogener Abschnitt;
- 26: Auflagefläche;
- 27: Drahtklammer;
- 28: Anschlag;
- 29: Auflagerippen;
- 30: Reinigungsspalt; und
- 31: Befestigungsausnehmung.

## Patentansprüche

1. Sterilcontainer (10) zur Aufbewahrung und zum Transport von medizinischen Utensilien mit einem stoffeinstückig mit einem geschlossenen, durchbruchfreien Abschnitt der Behälterwandung (11) des Sterilcontainers (10) ausgebildeten Befestigungsabschnitt (12), an welchem ein Anbauteil (20), welches aus einem sterilisationsbeständigen Material gefertigt ist, formschlüssig befestigbar oder federelastisch arretierbar ist, wobei eine Wandstärke des geschlossenen Abschnittes in der Behälterwandung (11), durch den der Befestigungsabschnitt (12) gebildet wird, im Wesentlichen konstant und gleich der Wandstärke der an diesen Abschnitt angrenzenden Behälterwandung (11) ist.

2. Sterilcontainer (10) gemäß Anspruch 1, **dadurch gekennzeichnet, dass** der Sterilcontainer (10) ein tiefgezogener Behälter mit im Wesentlichen einheitlicher Wandstärke ist.

3. Behältersystem mit einem Sterilcontainer (10) nach Anspruch 1 oder 2 zur Aufbewahrung und zum Transport von medizinischen Utensilien und dem an dem Sterilcontainer (10), insbesondere werkzeuglos, lösbar befestigbaren Anbauteil (20), welches aus dem sterilisationsbeständigen Material gefertigt ist,
**dadurch gekennzeichnet, dass**
das Anbauteil (20) an dem stoffeinstückig mit dem geschlossenen, durchbruchfreien Abschnitt der Behälterwandung (11) des Sterilcontainers (10) ausgebildeten Befestigungsabschnitt (12) an der Behälterwandung (11) formschlüssig befestigbar oder federelastisch arretierbar ist.

4. Behältersystem gemäß Anspruch 3, **dadurch gekennzeichnet, dass** das Anbauteil (20) zumindest ein Befestigungsglied (22) aufweist, welches am Anbauteil (20) ausgebildet und von einer Befestigungsstellung, in welcher es zum Ausüben des festlegenden Eingriffes mit dem entsprechenden Befestigungsabschnitt (12) ausgebildet ist, in eine Auslenkungsstellung, in welcher das Befestigungsglied (22) zum Überführen in den oder aus dem festlegenden Eingriff mit dem Befestigungsabschnitt (12) ausgelenkt und/oder verformt ist, überführbar ist.

5. Behältersystem gemäß Anspruch 3 oder 4, **dadurch gekennzeichnet, dass** das zumindest eine Befestigungsglied (22) oder das Anbauteil (20) als Ganzes zumindest abschnittsweise federelastisch ausgebildet und dazu angepasst ist, durch die interne Federwirkung des zumindest einen federelastischen Abschnitts das Befestigungsglied (22) aus der Auslenkungsstellung in die Befestigungsstellung zurückzuführen.

6. Behältersystem gemäß Anspruch 5, **dadurch gekennzeichnet, dass** der Befestigungsabschnitt (12) und/oder das Anbauteil (20) Führungsflächen (24) aufweisen, welche derart abgeschrägt oder abgerundet sind, dass durch Aufdrücken des Anbauteils (20) auf den Sterilcontainer (10) in eine Befestigungsrichtung, insbesondere eine Befestigungsrichtung im Wesentlichen senkrecht zu einer Behälterwand (13), das zumindest eine Befestigungsglied (22), unter Abgleiten der Führungsflächen (24) und Überwindung der internen Federwirkung, in die Auslenkungsstellung überführt wird.

7. Behältersystem gemäß einem der Ansprüche 5 oder 6, **dadurch gekennzeichnet, dass** das Anbauteil (20) zumindest zwei Befestigungsglieder (22) aufweist und der zumindest eine federelastische Abschnitt dazu angepasst ist, die Befestigungsglieder (22) in der Befestigungsstellung derart gegeneinander anzustellen, dass sie eine klemmende Wirkung mit dem Befestigungsabschnitt (12) entfalten.

8. Behältersystem gemäß einem der vorstehenden Ansprüche, **dadurch gekennzeichnet, dass** die Behälterwandung (11) zumindest abschnittsweise aus einem Blech aus medizintechnischem Metall gebildet wird und der Befestigungsabschnitt (12) durch Blechumformen eines solchen Blechabschnitts gebildet wird.

9. Behältersystem gemäß Anspruch 8, **dadurch gekennzeichnet, dass** der Befestigungsabschnitt (12) als zumindest eine kaltverformte Ausbuchtung oder Ausnehmung in den Behälter (10) eingebracht ist.

10. Behältersystem gemäß einem der vorgenannten Ansprüche 3 bis 9, **dadurch gekennzeichnet, dass** der Befestigungsabschnitt (12) an oder in einer einzigen Behälterwand (13) oder dem Deckel ausgebildet ist.

11. Behältersystem gemäß einem der Ansprüche 4 bis 10, **dadurch gekennzeichnet, dass** der Befestigungsabschnitt (12) zumindest eine, insbesondere zwei, gemeinsame Symmetrieebene mit dem oder den Befestigungsglied (22) aufweisen.

12. Behältersystem gemäß einem der der Ansprüche 4 bis 11, **dadurch gekennzeichnet, dass** das zumindest eine Befestigungsglied (22) einstückig mit dem Anbauteil (20) ausgebildet ist.

13. Behältersystem gemäß einem der vorgenannten Ansprüche 3 bis 12, **dadurch gekennzeichnet, dass** das Anbauteil (20) eine Klammer (27), insbesondere eine Drahtklammer, zum festklemmen eines Kennzeichnungsschildes aufweist.

14. Behältersystem gemäß einem der vorgenannten Ansprüche 3 bis 13, **dadurch gekennzeichnet, dass** das Anbauteil (20) Löcher oder Ausnehmungen zum Befestigen eines Kennzeichnungsschildes aufweist.

## Claims

1. A sterile container (10) for the storage and transport of medical utensils, having a fastening portion (12) which is formed in one piece of material with a closed portion free of apertures of a container wall (11) of the sterile container (10) and to which an attachment part (20), which is manufactured from a sterilization-resistant material, can be fastened in a positive-locking manner or can be locked in place in a spring-elastic manner, wherein a wall thickness of the closed portion in the container wall (11) is formed by the fastening portion (12) and is substantially constant and equal to the wall thickness of the container wall (11) bordering said portion.

2. The sterile container (10) according to claim 1, **characterized in that** the sterile container (10) is a deep-drawn container with substantially uniform wall thickness.

3. A container system which comprises a sterile container (10) according to claim 1 or 2 for storing and transporting medical utensils and includes the attachment part (20) which can be detachably fastened to the sterile container (10), in particular without using any tools, and which is made of the sterilization-resistant material,
**characterized in that**
the attachment part (20) can be positively fastened or resiliently locked in place on the container wall (11) on the fastening portion (12) formed in one piece of material with the closed portion free of apertures of the container wall (11) of the sterile container (10).

4. The container system according to claim 3, **characterized in that** the attachment part (20) has at least one fastening element (22) which is formed on the attachment part (20) and can be moved from a fastening position, in which it is designed for exerting the securing engagement with the corresponding fastening portion (12), to a deflected position, in which the fastening element (22) is deflected and/or deformed for moving it into or out of the securing engagement with the fastening portion (12).

5. The container system according to claim 3 or 4, **characterized in that** the at least one fastening element (22) or the attachment part (20) as a whole is formed to be resilient at least in sections and is adapted to return the fastening element (22) from the deflected position to the fastening position by the internal spring action of the at least one resilient portion.

6. The container system according to claim 5, **characterized in that** the fastening portion (12) and/or the attachment part (20) have guide surfaces (24) which are beveled or rounded in such a way that, by pressing the attachment part (20) onto the sterile container (10) in a fastening direction the at least one fastening element (22) is moved to the deflected position by sliding along the guide surfaces (24) and overcoming the internal spring action.

7. The container system according to one of claims 5 or 6, **characterized in that** the attachment part (20) has at least two fastening elements (22) and the at least one resilient portion is adapted to set the fastening elements (22) relative to one another in the fastening position in such a way that they exert a clamping action with the fastening portion (12).

8. The container system according to one of the preceding claims, **characterized in that** the container wall (11) is formed at least in sections from a sheet metal of medical grade metal and the fastening portion (12) is formed by sheet metal working of such a sheet metal section.

9. The container system according to claim 8, **characterized in that** the fastening portion (12) is worked into the container (10) as at least bulge or recess produced by work hardening.

10. The container system according to one of the preceding claims 3 to 9, **characterized in that** the fastening portion (12) is formed on or in a single container wall (13) or the lid.

11. The container system according to one of claims 4 to 10, **characterized in that** the fastening portion (12) has at least one, in particular two planes of symmetry in common with the fastening element(s) (22).

12. The container system according to one of claims 4 to 11, **characterized in that** the at least one fastening element (22) is formed integrally with the attachment part (20).

13. The container system according to one of the preceding claims 3 to 12, **characterized in that** the attachment part (20) has a clamp (27), in particular a wire clamp, for clamping an identification plate.

14. The container system according to one of the preceding claims 3 to 13, **characterized in that** the attachment part (20) has holes or recesses for fastening an identification plate.

## Revendications

1. Conteneur stérile (10) pour la conservation et le transport d'ustensiles médicaux avec une section de fixation (12) formée d'un seul tenant avec une section fermée, sans pénétration, de la paroi de conteneur (11) du conteneur stérile (10), sur laquelle une pièce rapportée (20), qui est constituée d'un matériau résistant à la stérilisation, peut être fixée par complémentarité de formes ou bloquée élastiquement, dans lequel une épaisseur de paroi de la section fermée dans la paroi de conteneur (11), par laquelle la section de fixation (12) est formée, est sensiblement constante et égale à l'épaisseur de paroi de la paroi de conteneur (11) adjacente à cette section.

2. Conteneur stérile (10) selon la revendication 1, **caractérisé en ce que** le conteneur stérile (10) est un conteneur embouti avec une épaisseur de paroi sensiblement uniforme.

3. Système de récipient avec un conteneur stérile (10) selon la revendication 1 ou 2 pour la conservation et le transport d'ustensiles médicaux et la pièce rapportée (20) pouvant être fixée au conteneur stérile (10) de manière amovible, en particulier sans outils, est constituée d'un matériau résistant à la stérilisation,
**caractérisé en ce que**
la pièce rapportée (20) peut être fixée à la paroi de récipient (11) par complémentarité de formes ou bloquée élastiquement au niveau de la section de fixation (12) formée d'un seul tenant avec la section fermée et sans pénétration de la paroi de récipient (11) du conteneur stérile (10).

4. Système de récipient selon la revendication 3, **caractérisé en ce que** la pièce rapportée (20) présente au moins un organe de fixation (22) qui est formé au niveau de la pièce rapportée (20) et peut être transféré d'une position de fixation, dans laquelle il est formé pour exercer l'engagement de fixation avec la section de fixation (12) correspondante, à une position de déviation dans laquelle l'organe de fixation (22) est dévié et/ou déformé pour être transféré dans ou hors de l'engagement de fixation avec la section de fixation (12).

5. Système de récipient selon la revendication 3 ou 4, **caractérisé en ce que** le au moins un organe de fixation (22) ou la pièce rapportée (20) dans son ensemble est formé élastiquement au moins par sections et est adapté pour ramener l'organe de fixation (22) de la position de déviation à la position de fixation par l'effet de ressort interne de la au moins une section élastique.

6. Système de récipient selon la revendication 5, **caractérisé en ce que** la section de fixation (12) et/ou la pièce rapportée (20) présentent des surfaces de guidage (24) qui sont biseautées ou arrondies de sorte qu'en emmanchant la pièce rapportée (20) sur le conteneur stérile (10) dans une direction de fixation, en particulier une direction de fixation sensiblement perpendiculaire à une paroi de récipient (13), le au moins un organe de fixation (22) est transféré dans la position de déviation en faisant glisser les surfaces de guidage (24) et en surmontant l'effet de ressort interne.

7. Système de récipient selon l'une quelconque des revendications 5 ou 6, **caractérisé en ce que** la pièce rapportée (20) présente au moins deux organes de fixation (22) et **en ce que** la au moins une section élastique est adaptée pour positionner les organes de fixation (22) l'un par rapport à l'autre dans la position de fixation de sorte qu'ils exercent une action de serrage avec la section de fixation (12).

8. Système de récipient selon l'une quelconque des revendications précédentes, **caractérisé en ce que** la paroi de récipient (11) est formée au moins par sections d'une tôle en métal médicotechnique, et la section de fixation (12) est formée par formage de tôle d'une telle section de tôle.

9. Système de récipient selon la revendication 8, **caractérisé en ce que** la section de fixation (12) est introduite dans le récipient (10) en tant qu'au moins un renflement ou évidement déformé à froid.

10. Système de récipient selon l'une quelconque des revendications 3 à 9 précitées, **caractérisé en ce que** la section de fixation (12) est formée au niveau de ou dans une seule paroi de récipient (13) ou le couvercle.

11. Système de récipient selon l'une quelconque des revendications 4 à 10, **caractérisé en ce que** la section de fixation (12) présente au moins un, en particulier deux, plans de symétrie communs avec le ou les organes de fixation (22).

12. Système de récipient selon l'une quelconque des revendications 4 à 11, **caractérisé en ce que** le au moins un organe de fixation (22) est formé d'un seul tenant avec la pièce rapportée (20).

13. Système de récipient selon l'une quelconque des revendications 3 à 12 précitées, **caractérisé en ce que** la pièce rapportée (20) présente une pince (27), en particulier une pince métallique, pour serrer une plaque d'identification.

14. Système de récipient selon l'une quelconque des revendications 3 à 13 précitées, **caractérisé en ce que** la pièce rapportée (20) présente des trous ou des évidements pour la fixation d'une plaque d'identification.
